# DEMANDE DE BREVET EUROPEEN

(11) **EP 3 960 208 A1**
(43) Date de publication de la demande: **02.03.2022**
(21) Numéro de dépôt: 21189480.3
(22) Date de dépôt: 03.08.2021
(51) Int. Cl.: A61L 2/10, A61L 2/26

(54) **ENCEINTE DE STÉRILISATION UVC**

(30) Priorité: 27.08.2020 BE 202005594
(71) Demandeur: Twylite, 1000 Bruxelles (BE)
(72) Inventeur: VERSTRAETE, Bruno, 1000 Bruxelles (BE)
(74) Mandataire: Office Kirkpatrick

(57) **Abrégé**

L'invention concerne une enceinte étanche de décontamination munie d'une porte et à l'intérieur de laquelle sont agencées des LED UVC programmées pour s'allumer durant une durée prédéterminée uniquement lorsque la porte est fermée. L'enceinte de l'invention permet d'utiliser, sans risque pour l'utilisateur les UVC pour décontaminer rapidement une large de gamme d'objets et est efficace aussi bien contre les contaminations bactériennes que virales.

## Description

L'invention se situe dans le domaine de la stérilisation ou décontamination d'objets par application d'UVC, dans une enceinte fermée présentant toutes les garanties de sécurité liée à l'utilisation d'UVC.

### Arrière plan de l'invention

Les UVC (rayons ultra-violet C) sont connus pour leur pouvoir désinfectant, aussi bien sur les bactéries que sur les virus, alors que les UVA, les plus souvent utilisés n'ont aucun effet sur les souches virales. Les UVC agissent pas modification de la structure de l'ADN et de l'ARN et présentent donc également un risque pour la santé humaine.

Les UV-C sont définis par leur longueur d'onde allant de 100 nm à 285 nm, la plage de longueur d'onde généralement associée à la désinfection étant généralement comprise plutôt entre 200 et 285 nm.

En raison de sa dangerosité, l'utilisation de sources d'UVC est fortement réglementée et la mise en œuvre de solutions de désinfection impliquant des UVC est particulièrement complexe. Les UVC sont par exemple utilisés comme purificateur d'air dans des conduits d'aération, là où aucun rayon ne peut atteindre un humain. Ils sont utilisés dans certaines contextes professionnels sensibles, comme des hôpitaux ou des laboratoires de biologie, où leur utilisation est réservée à du personnel formé.

Durant la pandémie de Covid-19, un réel besoin de moyens d'élimination des virus a émergé, tant chez les particuliers que chez les professionnels : décontamination de matériel de travail utilisés par plusieurs personnes, décontamination d'articles manipulés mais non achetés par les clients dans les commerces, décontamination de livres dans les bibliothèques, etc... Les solutions généralement adoptées sont de nettoyer ces articles avec des solutions désinfectantes ou de mettre de côté ces articles pendant plusieurs jours avant de les rendre à nouveau accessible.

Il en résulte un cout considérable pour les commerçants et les professionnels, et un impact écologique non négligeable (matériel de désinfection jetable).

La demanderesse a jugé utile de proposer une solution de décontamination plus rapide et sure d'utilisation.

### Solution de l'invention

A cette fin, l'invention concerne une enceinte étanche de décontamination munie d'une porte et à l'intérieur de laquelle sont agencées des LED UVC programmées pour s'allumer durant une durée prédéterminée uniquement lorsque la porte est fermée. Une enceinte désigne ici un espace restreint, Il peut s'agir d'un élément de type mobilier, comme un placard, ou, de préférence, un élément facilement transportable, i.e. dont les dimensions et le poids (<20kg) permettent de la porter et de la mettre dans un véhicule, comme par exemple une caisse, une boite, un coffre, une malle. Il ne s'agit pas d'une caisse en carton, mais d'une enceinte rigide, réutilisable, par exemple une caisse en plastique moulé.

L'enceinte est étanche ou hermétique à la lumière. Cela implique généralement que l'enceinte est également étanche à l'air et à l'eau (norme IP 65).

L'enceinte est munie d'une porte au sens large d'une ouverture obturable. Si l'enceinte est de type caisse de transport, la porte est alors un couvercle. Le couvercle peut s'ouvrir par rotation autour de charnières.

Des LED UVC sont des diodes électroluminescentes émettant de la lumière dans les longueurs d'onde UVC, de préférence entre 200 et 285 nm, de préférence entre 240 et 260 nm.

Les LEDs UVC de l'invention sont de préférence des LEDs carrées. Elles peuvent émettre selon un angle de 135°.

Contrairement aux autres sources d'UVC, les LEDs ont l'avantage de ne pas émettre d'ozone.

Les LEDs UVC sont agencés à l'intérieur de l'enceinte de façon à irradier le maximum de surface des objets placés dans l'enceinte lorsque les LED sont allumées. De préférence, les parois intérieures de l'enceinte sont au moins en partie recouvertes d'un revêtement réfléchissant pour renvoyer vers le contenu de l'enceinte un maximum de lumière UVC.

Dans le cas d'une enceinte de type caisse, le fond de l'enceinte est de préférence pourvu d'un double fond surélevé transparent aux UVC pour que même la surface des objets à décontaminée qui est orientée vers le fond puisse être irradiée.

Les LED étant des composants fragiles de petite taille, plusieurs LED sont de préférence implantées sur une barrette, de préférence en série, da façon bien connue de l'homme du métier.

Les LEDs et l'installation électrique qui les pilote dégagent de la chaleur qui doit être absorbée dans l'enceinte. Etant donné qu'il n'est pas possible d'utiliser de ventilateur dans l'enceinte étanche, les LEDs sont de préférence implantées sur une barrette en MCPCB (Metal Core PCB), associée à un dissipateur thermique.

Les LEDs sont intégrées dans un circuit électrique de contrôle de leur allumage sous certaines conditions.

Avantageusement, dans le cas où l'enceinte est une caisse, le circuit électrique et les LEDs peuvent être disposées dans le couvercle de la caisse, l'irradiation se fait alors par le haut de l'enceinte et profite des parois réfléchissantes de la caisse pour irradier toutes les faces des objets à décontaminer, qui sont de préférence placés surélevés par rapport au fond de la caisse.

Les LEDs sont programmées pour s'allumer durant une durée déterminée prenant en compte notamment la dimension de l'enceinte et la puissance totale des LEDs. La durée prédéterminée représente un cycle de décontamination. De préférence, l'enceinte (dimension et puissance/nombre des LEDs) est agencée pour que le cycle de décontamination dure moins de trente minutes, de préférence moins de 15 minutes pour une efficacité supérieure ou égale à 99.99%.

La lumière UVC étant invisible pour l'homme mais dangereuse pour lui, les LEDs sont programmées pour s'allumer uniquement lorsque la porte est fermée. En particulier, l'enceinte comprend des moyens de détection de fermeture de la porte agencés pour contrôler l'allumage des LEDs, l'allumage des LEDs n'étant permis que lorsque la porte de l'enceinte est fermée. Ces moyens de détection de fermeture sont de préférence des moyens mécaniques permettant d'établir une connexion électrique lorsque la porte est fermée. L'allumage des LEDs peut être conditionné à d'autres facteurs, notamment le déclenchement du cycle de décontamination par un utilisateur.

L'invention sera mieux comprise à l'aide de la description suivante en référence au dessin sur lequel :

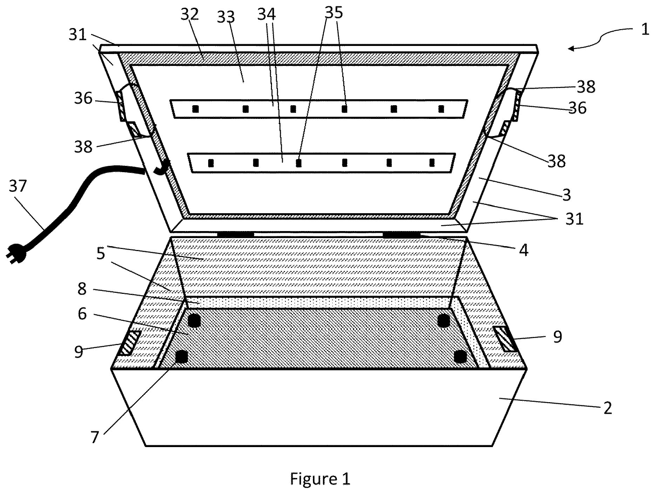

La figure 1 est un schéma d'une enceinte selon l'invention.

Dans un mode de réalisation, illustré sur la figure 1, une enceinte selon l'invention est une caisse 1, de forme générale parallélépipédique, par exemple ici une caisse de transport de matériel audio-vidéo. La caisse est composée d'un bac 2, constituant le principal volume de la caisse, et un couvercle 3. Le couvercle 3 et le bac 2 sont attachés par deux charnières 4 disposées ici le long d'un bord de la caisse et du bord correspondant du couvercle de façon à ce que le couvercle pivote autour des charnières et recouvre, en position fermée, toute la caisse. Il s'agit d'une caisse classique, pouvant être fermée de façon totalement étanche. La caisse est par exemple en plastique moulé. Elle peut être équipée d'une poignée (non ici représentée). Des moyens de fermeture et de verrouillage de la caisse, i.e. pour maintenir le couvercle et le bac fermés de façon étanche, sont également présents (non ici représentés), de préférence au moins le long du bord opposé aux charnières.

Les parois intérieures 5 du bac sont couvertes d'un revêtement réfléchissant, ainsi que le fond 6 du bac. Quatre plots 7 sont disposés au fond du bac. Une plaque 8 de plexiglas transparent aux UVC est posée sur ces plots 7. Deux plaquettes métalliques 9 de contact sont disposés en haut des bords latéraux du bac, parallèles au fond 6.

LE couvercle 3 est ici un couvercle creux, c'est-à-dire définissant un volume grâce à quatre rebords 31 s'étendant autour du fond 32 de couvercle perpendiculairement à ce fond 32. Une plaque 33, de surface légèrement inférieure à celle du fond de couvercle 32 est fixée dans le couvercle 3. La plaque 33 n'est pas plaquée contre le fond du couvercle mais est fixée de façon à laisser un espace entre la plaque 33 et le fond 32. La plaque 33 a ainsi une face visible et une face cachée (tournée vers le fond du couvercle). Sur la face visible de la plaque 33 sont fixées, parallèlement dans la longueur de la plaque, deux barrettes 34 le long desquelles sont implantées, sur chaque barrette 34, six LEDs UVC 35, ici des LEDs carrées d'intensité 650 mA, émettant entre 242 et 258 nm sur une plage angulaire de 135°, reliées en série par des pistes en cuivre.

Chaque barrette est reliée à une unité de contrôle placée ici entre la plaque 33 et le fond du couvercle (ici non représenté). Un dissipateur thermique est également installé en regard de chaque barrette 34, sur la face cachée de la plaque 33. Il s'agit par exemple d'un dissipateur thermique à lamelles, dont la longueur et la largeur sont sensiblement les mêmes que celles des barrettes, ou supérieures, et qui sont disposées parallèlement aux barrettes, de l'autre côté de la plaque métallique.

Un actuateur ou actionneur 36 est placé sur chaque rebord latéral du couvercle, au même niveau que la plaquette 9 sur le bord latéral du bac. Les actuateurs 36 sont reliés par des fils 38 à l'unité de contrôle comprenant notamment une minuterie.

Un câble l'alimentation 37 traverse un rebord du couvercle pour relier l'unité de contrôle à une fiche de connexion située à l'extérieur de la caisse.

Pour éviter qu'un utilisateur ne touche les LEDs, qui sont fragiles et sensibles à l'électricité statique, une plaque de plexiglas transparent aux UVC est de préférence placée devant la plaque 33 de support des LEDs. Ainsi, l'accès à l'électronique est protégé sans empêcher les rayons UVC d'irradier l'intérieur du bac.

Les éléments de la caisse de l'invention ayant été décrit, son fonctionnement va maintenant être détaillé.

Un objet à désinfecter, par exemple une caméra vidéo, est posé dans le bac 2 sur la plaque 8 de plexiglas transparent. Il ne touche ainsi pas le fond du bac.

Le couvercle 3 est rabattu sur le bac 2 par rotation autour des charnières. Lorsque que la caisse est totalement fermée, et de préférence verrouillée grâce à au moins deux loquets, les acuateurs 36 appuient sur les plaquettes 9 de façon à fermer le circuit électrique. L'utilisateur va brancher le câble d'alimentation 37 et éventuellement appuyer sur un bouton de démarrage du cycle de décontamination.

L'unité de contrôle déclenche alors l'allumage des LEDs pendant une durée prédéterminée, par exemple quatre minutes. Cette durée a été prédéterminée en fonction du volume de la caisse, du nombre et le la puissance des LEDs. Par exemple, une quantité d'énergie de 75 mJ/cm² de surface à décontaminer sont suffisant pour tuer 99.99% de virus de type Covid-19, 140 mJc/m² sont nécessaire pour le même effet sur le virus SARS. De préférence, la puissance lumineuse apportée est supérieure à la puissance théorique requise, par exemple 30% supérieure.

A la fin du cycle, l'unité de contrôle éteint les LEDs et la caisse peut être à nouveau ouverte.

Si la caisse est ouverte pendant le cycle, celui s'arrête automatiquement, les actuateurs n'étant plus en contact avec les plaquettes 9, le circuit étant alors ouvert. Les actuateurs sont donc des sécurités permettant qu'en cas de mauvaise utilisation de la caisse, aucun rayon UVC ne puisse jamais atteindre un humain.

Durant le cycle, les rayons UVC irradient toute la surface de l'objet à décontaminer, grâce notamment à la réflexion des rayons sur les parois réfléchissantes du bac 2 et sur le fond. L'objet étant surélevé par rapport au fond, Les UVC peuvent même l'irradier par en dessous. La plaque 33 dans le couvercle est avantageusement en aluminium, ce qui permet également de réfléchir les rayons.

Les LEDs ne génèrent pas d'ozone qui pourrait endommager la caméra vidéo, comme par exemple les membranes de micro.

Les dissipateurs thermiques couplés aux barrettes de LEDs permettent de limiter l'élévation de température, sans recourir à un ventilateur. Typiquement, la température ne dépasse pas 47 °C.

Les barrettes de LEDs sont de préférence reliées à la masse pour éviter toute formation d'électricité statique.

Tous les composants de la caisse sont de préférence certifiés résistants aux UVC, pour assurer la durabilité de la caisse. Ils sont de préférence certifiés IP65.

Ici, l'alimentation électrique est assurée par branchement sur secteur du câble d'alimentation. Ce câble traverse le rebord du couvercle par un orifice équipé d'un joint étanche. L'alimentation électrique pourrait également se faire à l'aide de batteries rechargeables installées sur la caisse.

L'unité de contrôle comprend une minuterie, par exemple un relais temporisé, et est configurée pour ne déclencher l'allumage des lampes que si la caisse est fermée hermétiquement, c'est-à-dire, ici, que le contact entre deux lamelles des actuateurs soit assuré par pression de ces lamelles contre les plaquettes 9. Tout autre système de sécurité équivalent est envisageable, l'essentiel étant que les LEDs s'éteignent dès que l'étanchéité de la caisse est rompue.

Les barrettes de LEDs contiennent ici six LEDs chacune, mais elles peuvent en contenir plus, par exemple douze ou dix-huit, selon la dimension de la caisse. De même il peut y avoir une ou plusieurs barrettes, par exemple trois, quatre ou même plus.

La caisse permet de décontaminer une grande variété d'objets et a, par exemple, été testée avec du matériel audio-visuel, des smartphones, des batteries, des ordinateurs, des bouteilles, du maquillage..., sans qu'une détérioration de ces objets ait été constatée. La caisse peut également être utilisée par exemple pour décontaminer des vêtements, de l'argent, des masques, du matériel médical, ou des produits cosmétiques, ou tout autre objet résistant au cycle de décontamination par UVC mis en œuvre dans la caisse.

Une caisse a ici été décrite, dans laquelle les LEDs et l'électronique qui les pilote est installée dans le couvercle. Néanmoins, une autre configuration pourrait être envisagée, les LEDs et leur électronique pourraient être disposées dans le fond, pour alléger le couvercle et maintenir le centre de gravité de la caisse bas.

Les LEDs et leur électronique pourraient également être placées contre une paroi latérale, ou couvrir plusieurs parois de l'enceinte.

Lorsque l'enceinte n'est pas une caisse mais a, par exemple, une forme de placard avec un porte verticale, la paroi du fond du placard peut être la paroi équipée des LEDs. Cela est particulièrement intéressant, par exemple pour la décontamination de vêtements, après que ceux-ci aient été essayé par des clients sans être achetés. Le commerçant peut alors remettre rapidement les vêtements en rayons après un cycle de décontamination.

## Revendications

1. Enceinte étanche de décontamination munie d'une porte et à l'intérieur de laquelle sont agencées des LEDs UVC programmées pour s'allumer durant une durée prédéterminée uniquement lorsque la porte est fermée.

2. Enceinte selon la revendication 1, ladite enceinte étant une caisse de transport dont la porte est un couvercle.

3. Enceinte selon la revendication 2, dans laquelle les LEDs UVC sont logées dans le couvercle.

4. Enceinte selon l'une des revendications 1 à 3, étanche à la lumière.

5. Enceinte selon l'une des revendications 1 à 4, dans laquelle les parois intérieures sont au moins en partie recouvertes d'un revêtement réfléchissant.

6. Enceinte selon l'une des revendications 1 à 5, comprenant un double fond surélevé transparent aux UVC.

7. Enceinte selon l'une des revendications 1 à 6, dans laquelle les LEDs sont implantées sur une barrette en MCPCB.

8. Enceinte selon l'une des revendications 1 à 7, dans laquelle les LEDs sont associée à au moins un dissipateur thermique.

9. Enceinte selon l'une des revendications 1 à 8, dans laquelle les LEDs UVC éclairent à une longueur d'onde comprise entre 240 nm et 280 nm.

10. Enceinte selon l'une des revendications 1 à 9, comprenant des moyens des moyens de détection de fermeture de la porte agencés agencés pour contrôler l'allumage des LEDs.

11. Enceinte selon la revendication 10, dans laquelle les moyens de détection de fermeture sont des moyens mécaniques permettant d'établir une connexion électrique lorsque la porte est fermée.

12. Utilisation de l'enceinte selon l'une des revendications 1 à 11 pour éliminer ou réduire les virus sur des objets placés dans l'enceinte.

13. Utilisation de l'enceinte selon la revendication 13 pour décontaminer du matériel audio-visuel, des vêtements, de l'argent, des masques ou des produits cosmétiques.
